# EUROPEAN PATENT APPLICATION

(11) **EP 1 043 032 A2**
(43) Date of publication of application: **11.10.2000**
(21) Application number: 00109341.8
(22) Date of filing: 26.06.1995
(51) Int. Cl.: A61L 2/06, A47L 11/34, A01N 25/00

(54) **Use of moisture and heat for de-naturing house dust mite (HDM) allergen**

(30) Priority: 29.07.1994 GB 9415317; 20.09.1994 GB 9418933; 28.03.1995 GB 9506289
(62) Divisional of application: 95922669.7
(71) Applicant: Houlbrook, Kenneth, Pateley Bridge HG3 5BN (GB)
(72) Inventor: Houlbrook, Kenneth, Pateley Bridge HG3 5BN (GB)
(74) Representative: Denmark, James Christopher

(57) **Abstract**

The invention provides that HDM (House Dust Mite) and its allergens can be treated in the breeding grounds in the home (fabrics, seats, chairs, mattresses, pillows, sheets, blankets, cushions, etc) by steam treatment followed by hot air drying. The invention also discloses that these articles can be specially adapted to receive an injection device so that the steam and hot air can be injected deep into the article core so as to penetrate outwardly to the surface to treat and heat the entire article to a high allergen denaturing temperature.

## Description

This invention relates to the de-naturing of the HDM allergens, for the improvement of living conditions for asthma and the like sufferers, and the invention achieves this in various ways, involving methods, articles and equipment. The invention represents a breakthrough in relation to the problem that the HDM allergens are the major cause of discomfort to asthma and the like sufferers as evidenced by medical studies (See M J Bluff "Use of Liquid Nitrogen in the Control of House Dust Mite Population" - Clinical Allergy, 1986 - Volume 16, pages 41 to 47).

The present invention has particular application to the treatment of articles such as those containing textiles, in particular voluminous soft furnishings such as mattresses, bed bases, pillows, sofas, cushions, soft toys and the like which constitute breeding grounds for the HDM. The treatment to which the invention relates is one whereby the HDM (Dermatophagoides Patheronyssinus) may be killed, and allergen contained in its droppings may be de-natured.

The HDM, as its name suggests, inhabits indoor environments, in particular those inhabited by humans or animals. Amounts of microscopic flakes of skin are shed continually by humans or animals, and these flakes fall to the floor and onto furnishings in a household, and where there is a fabric-covered article, the skin flakes will gradually seat within the article. The HDM feeds from these skin flakes as they decompose, and it will be appreciated that where large amounts of skin are collected, large concentrations of HDM are found. Textiles are particularly susceptible to infestation by HDM since skin particles collect in the spaces between the fibres of the textile, in whiqh spaces the HDMs are able to live and reproduce generally without excess hindrance. Bedding materials are particularly infested with HDMs since large amounts of skin are shed in beds, onto bed bases and duvets, and HDM concentrations build up unchecked.

Research has shown that the effects of living in environments free of HDMs, such as hospitals, where few places are found for the HDM to live and breed, is often beneficial to humans with a propensity to develop respiratory problems such as asthma and other diseases such as rinitis and eczema. It is believed that not the HDM itself, but an allergen or group of allergens called Der.p1 (herein "HDM allergen"), associated with the faecal pellets of the mites, is a contributing factor if not a major cause of these diseases. It is believed that the HDM allergen causes an allergic reaction which brings about inflammation of tissue at the surface of the broncheoles in the lungs of an asthmatic person where the allergen comes to rest, where the inflammation causes breathing difficulties.

Various methods and means have been proposed to eliminate the HDM from bedding materials belonging to asthma sufferers in particular.

In the home there are several forms of textile article which form infestation grounds for the HDM and these are two-dimensional type sheet materials such as curtains, carpets, upholstery coverings and the like, which are difficult to clean, and three-dimensional textile articles such as pillows, cushions, mattresses, chairs, sofas, soft toys and so on, and intermediate types of articles such as duvets and the present invention in its various aspects addresses all types of article. As a major improvement relates to the treatment of the so-called three-dimensional article, much of the discussion herein relates to that treatment.

Some two-dimensional textile articles, such as clothes and bedclothes may be washed to eliminate the HDM and its allergen, but same the articles containing a textile which are treated by the invention are obviously not susceptible to such treatment.

There have been attempts to provide treatment of and machines for treating textile articles for the elimination of the HDM and its allergens. There have also been proposed machines for the cleaning of the two-dimensional articles such as carpets by treatment which may involve the unwitting killing of the HDM and the de-naturing of the allergens but the prior proposals have failed to provide a completely effective solution to the problem, by means of a quick and efficient treatment (which can be performed as a service to, or can be performed by a householder) which leaves the treated article completely or substantially free of HDM and de-naturing the HDM allergens leaving the article in an HDM free condition after treatment.

Thus, there are known vacuum cleaners specially adapted for the removal of HDM from two-dimensional fabric articles, the cleaners having powerful suction and fine filtering arrangements. In one specially adapted vacuum cleaner, once the dust has been vacuumed and collected, heat is applied to the collecting receptacle at 60°C to kill the HDMs contained in the dust. These vacuum cleaners are not suitable for use in the cleaning of three-dimensional textile articles; the cleaner cannot apply the pressure required to effectively remove the embedded allergen particles, nor the large numbers of mites which remain in place by clinging onto the textile fibres. Our research furthermore has shown that 60°C is an insufficient temperature to de-nature the allergen in a reasonable time.

A further product marketed for use in conjunction with the abovementioned vacuum cleaners is that of a pesticide spray which kills the HDM. Many people are however wary of using pesticide sprays, particularly in the household. Large amounts of the spray would be required in order to treat, for example, a single mattress. Furthermore, the spray may kill the HDMs, but the allergen is not affected and remains potent.

Another proposed method of preventing HDM from building up and the HDM allergen to become airborne is that of covering the bed with an air-impermeable sheet of plastic. However in general such plastic coverings have been found to reduce the levels of comfort achieved by the use of textile coverings only.

In British Patent Application No. 2280851, it has been proposed to use steam to de-nature the HDM allergen, but that patent application merely suggests the use of steam without giving further particulars for a complete and effective treatment. A drawing in the application shows what appears to be a steam generating machine and an outlet nozzle jetting steam into a floor covering, but no description is given in relation thereto. This patent application was not published until 15th February 1995.

British Patent No. 483710 disclosed the same basic equipment for parasite diminution.

European Patent No. 0395781 B1 discloses a method of combating the HDM by means of a vacuum cleaner suction nozzle co-operating with a hot air source, which comprises a hot air jet at 60°C to 70°C directed to the textile material to be treated. Whilst this hot air may kill the HDM, it does not at least within a sensible period of time de-nature the HDM allergen. The patent in any event does not recognise the need to kill the HDM allergen and makes no reference thereto.

European Patent Application No. 0424070 A1 describes one example of the use of a spray of treatment medium i.e. liquid nitrogen, for the killing of the dust mite; the application makes no disclosure concerning the de-naturing of the HDM allergen.

British Patents Nos. 1286985, 1448434 and 1520761, and US Patent 3262146 disclose that carpets and the like may be cleaned by dry, super heated steam jets followed by vacuum application.

There has been therefore much attention to the need to kill the HDM, but less attention to or realisation of the need to de-nature the HDM allergen and whilst this may take place in some of the prior art treatment by design or by accident, this realisation has contributed to the present invention, at least in some of its aspects.

This invention also recognises the need to provide a practical, quick and efficient treatment which can be carried out leaving the articles treated ready for use in as short a time as possible.

Thus, our research has indicated that depending upon the treatment not only does heat need to be present to denature the HDM allergen, but moisture must also be present, if temperature and treatment time are to be kept to reasonable, practical levels. We believe the article must be at a sufficiently high temperature (80°-100°C) dependent upon time to de-nature the allergen and/or dry the article. In atmospheric conditions we have found that although the steam is jetted from a vessel in which it is contained at above atmospheric pressure, when it is released, the article cannot be heated to over 100°C. Fortunately, this is high enough to de-nature the HDM allergen and at the same time low enough to avoid damage to the article.

Also, in relation to another aspect of the invention, by virtue of the particular method of and means for applying heat to the article, it is possible that heat may be applied without actually using moisture, even although a somewhat less effective method may result. For example, in this aspect of the invention, hot air at or sufficiently high temperature may be used and this will kill the HDM. If the HDM are effectively and regularly killed by this method, eventually the allergen will reduce.

Therefore, it is an object of the present application in at least some aspects, to provide a method of treating HDM containing articles whereby a large proportion of the HDMs are eliminated without the drawbacks and the inefficiencies of the known methods.

A further object of the invention at least in some aspects is the killing of the HDM and by natural means which leave no harmful residues.

It is a yet further object of the invention at least in some aspects to provide a method of destroying or de-naturing insitu the HDM allergen which collects in the articles.

According to a first aspect of the present invention, there is provided a method for the de-naturing of the HDM allergens in materials and articles which are breeding grounds for the HDM, characterised by:-
(i) rendering the material or article moist and heating same for a sufficient time to de-nature the allergens;
(ii) and including forced drying of the material or article to drive off the residual moisture remaining from step (i).

The presence of heat and moisture is, we have found, a major key in effectively de-naturing the HDM allergens. The heat and moisture may be applied in various ways. Thus, the material or article may be treated with cold water and then force dried, the drying step providing the heat; the material may be treated with hot water, the heat in the water providing the heat or some of it providing the means to de-nature the allergen, and the drying step either driving off the residual moisture or contributing to the supply of heat to de-nature the HDM allergen; the material may be treated with steam which supplies the moisture and heat, the drying step driving off residual moisture.

The important aspect therefore is that moisture and heat should be present, and residual moisture should be driven off by a drying step, which may or may not involve heat. The temperature should be sufficiently high to enable the treatment to be effective in a reasonable period of time e.g. no longer than two hours from start to finish and preferably considerably less than two hours.

The article to be treated may be pre-heated for example by hot air, before the application of moisture and the moisture may be provided in the carrier of a treatment chemical to enhance the process.

The article or material should be heated to a sufficient temperature and for a sufficient time in order to kill a substantial proportion of the HDMs. Preferably, the article or material is heated to at least 60°C. The time required will reduce as the temperature achieved increases.

Many soft furnishings contain fabric of synthetic materials such as nylon, and it will often be essential that the article is not irreperably damaged during the treatment. It has been estimated that a maximum "safe" final temperature for the article or material undergoing treatment may be up to 120°C or sometimes even 150°C, in order to obviate damage to the least heat-resistant materials.

An optimum temperature may be of the order of 100°C, which if maintained over a period of say ten minutes will ensure that the majority of HDMs are killed and the majority of the allergen material will be de-natured, whilst avoiding harm to heat sensitive textile materials.

Steam will preferably be used, which supplies both moisture and heat to the fibrous material. Steaming the article or material to become moist during treatment and the forced drying after the moisture treatment is an important step in this aspect of the present invention, as the treatment can be effected quickly and effectively. The article might also suitably be force heated before steaming by the application of heated air.

Also, according to the invention there is provided a method for the de-naturing of the HDM allergens in articles, such as pillows, cushions, mattresses, divans, duvets, chairs, settees, soft toys and the like, which are breeding grounds for the HDM, and which have a core, characterised by the step of heating the article to a sufficient temperature to de-nature the HDM allergen, by heating from the core outwardly.

In this aspect of the invention, the heating of the article from the "core", any heating means can be used, and although the use of steam provides the same advantages as described above, no prior art method has suggested heating from the core to ensure that effective heating of the article throughout its thickness takes place and when the temperature of the article at its surface reaches the desired temperature, effective heating has been completed.

As will be explained herein, the first and second aspects of the invention preferably are combined.

If the article is covered with a fabric outer layer, such as is the case with mattresses, heated fluid may be injected into the core of the article to heat through the fabric covering of the article by the use of an applicator head connected to a hot gas blower, the head having a lever which projects into the article.

When a heating apparatus uses a steam heat exchanger to provide hot air by heat exchange between the heat exchanger coils through which steam passes, such apparatus is particularly adaptable for the present invention, in that the steam circuit can be provided with a value for the selective bleeding of steam therefrom, whereby there is a supply of hot air on the one hand and a supply of steam on the other hand which can be used selectively for applying moisture and heat (steam supply) and hot air on the other hand for the forced drying step as indicated above.

Such an apparatus may comprise a treatment apparatus for cleaning carpets and other fabrics in domestic and industrial situations and which comprises a portable heat including outlets for steam, and hot air, and optionally vacuum, connected by appropriate piping, for example to a generating vehicle provided with a steam generator, blower and vacuum generator.

In another apparatus, which may be a tumble drier apparatus, having a steam driven heat exchanger, the steam can be supplied selectively, manually or automatically, into the drying chamber for the moisture/hot air drying treatment of the articles for the de-naturing of the HDM allergen.

Where the article, as in the case of conventionally sprung mattresses, contains a sizable internal cavity surrounded by a textile covering, the cavity may be utilized in order to distribute the heated fluid throughout the interior of the article, and the applicator conduit may be used to transmit the heated fluid directly into the cavity to enable a full permeation of the heated fluid through the textile. The applicator conduit may be in the form of an apertured conduit located to lie along the length of the mattress, the apertures allowing release of the heated fluid at regular intervals along its length.

Even when the article has no large internal cavity, for example as in the case of a pillow, seat or cushion, homogenous permeation of heated fluid throughout the article using a single lance may still be affected. If the article has multiple cavities, in order to heat the fabric, a number of applicator conduits or lances may be inserted to lie with outlets distributed regularly throughout the article and/or textile. Each such conduit preferably comprises a number of outlets along its length internally of the article.

The article may be externally insulated during the permeation of the heated fluid in order to reduce the the energy and time required in order to effect treatment, thereby making the process more efficient. The article may suitably be wrapped in an insulating foam rubber covering. The covering may be specially adapted to receive the article snugly, with the covering having an aperture allowing the insertion of the applicator lance or lances.

When so insulated, and pressurised heating fluid is delivered to the article to heat same, the fluid may be recirculated to conserve energy and promote the permeation of the heated fluid throughout the fabric of the article.

In a further embodiment of this aspect of the invention, a mattress or other article containing a fabric may be provided with a pre-fitted heating element or hot gas blower which is adapted to cause heating of the article from the core by the permeation of heated air troughout the fabric of the article to suitably elevate the temperature thereof for a period of time in order to de-nature the HDM allergen and/or the HDM contained therein.

Also according to the invention, in a third aspect, there is provided an article such as a pillow, cushion, mattress, divan, duvet, chair, settee, soft toy and the like, which is a breeding ground for the HDM and which has a core, characterised in that the article is adapted to be treated by being heated, by hot fluid medium for the de-naturing, at periodic intervals, of the HDM allergen, said adaptation comprising providing the article with an integral coupling means for an injection device by which the fluid or fluids which perform the de-naturing can be injected into the core of the article.

Embodiments of the various aspects of the invention will now be described, by way of example, with reference to the diagrammatic drawings, wherein:-
Fig. 1 is an illustration showing a method of treatment according to a first aspect of the invention;
Fig. 2 is a view similar to Fig. 1, but showing slightly modified equipment;
Fig. 3 is a view similar to Fig. 1f but showing an apparatus according to a further embodiment of the present invention;
Fig. 4 is a sectional view of an apparatus according to a still further embodiment of the invention;
Fig. 5 is an enlarged view showing a method of treatment according to a second aspect of the invention, the article being treated being a mattress;
Fig. 6 is a view similar to Fig. 5 but showing the treatment as applied to a pillow;
Fig. 7 is a perspective view of a pillow showing an article according to a third aspect of the invention;
Fig. 8 is a plan view of an equipment usable in the methods shown in Figs. 5 and 6; and
Fig. 9 is a further enlarged view of an injection lance suitable for use in the methods of the invention shown in Figs. 3 and 4.

The present invention in all of its aspects is concerned with the treatment of materials and articles which form breeding grounds for the HDM. The invention in its aspects addresses the need on the one hand to eliminate the HDM, and on the other hand to de-nature the HDM allergens. In a first aspect of the invention, direction is given to the de-naturing of the HDM allergens insofar as the method involves the use of moisture, preferably hot water or steam, because according to our research work, it has been discovered, at least at the temperatures used in the tests, that moisture must be present for the effective de-naturing of these allergens. All of the aspects of the invention involve the application of heat, and at the temperatures which we were able to achieve in practise for heating the materials and articles and using steam, the presence of moisture is necessary to de-nature the allergens. In practise using steam, tests showed that it was difficult to heat any material or article to a temperature above 100°C.

In the second aspect of the invention, basically the same objective is involved, i.e. treatment of articles which form sites for HDM infestation, for the elimination of the HDM, but the second aspect involves a method of treatment which has not heretofore been suggested in the prior art, and in this method utilisation of heat alone will provide an advantageous effect. It is of course preferred that water be used for the effective de-naturing of the allergens, but the second aspect of the invention is not limited in this way. The third aspect of the invention concerns articles which are specifically adapted to enable treatment to be effected thereon. The concept is that these articles will be used in place of the conventional articles such as pillows and mattresses, but will be so designed that they can readily be treated simply by bringing the treatment equipment to the location of the article and by coupling it to the article by the special adaptation. For example the bedroom of an asthma sufferer may be provided with a specially adapted mattress and pillow which can be heated frqm the core at will by the coupling of injection apparatus for injecting heating fluid into the core of the mattress and pillow. If this is done on a regular basis, and the method may be according to the first aspect of the invention, the effective HDM removal and HDM allergen de-naturing will take place.

The invention further provides specifically adapted apparatus which is particularly effective in specific applications.

All of the aspects of the invention are inter-related and can be used in conjunction with one another as will be understood from the following more specific description of various embodiments of these aspects.

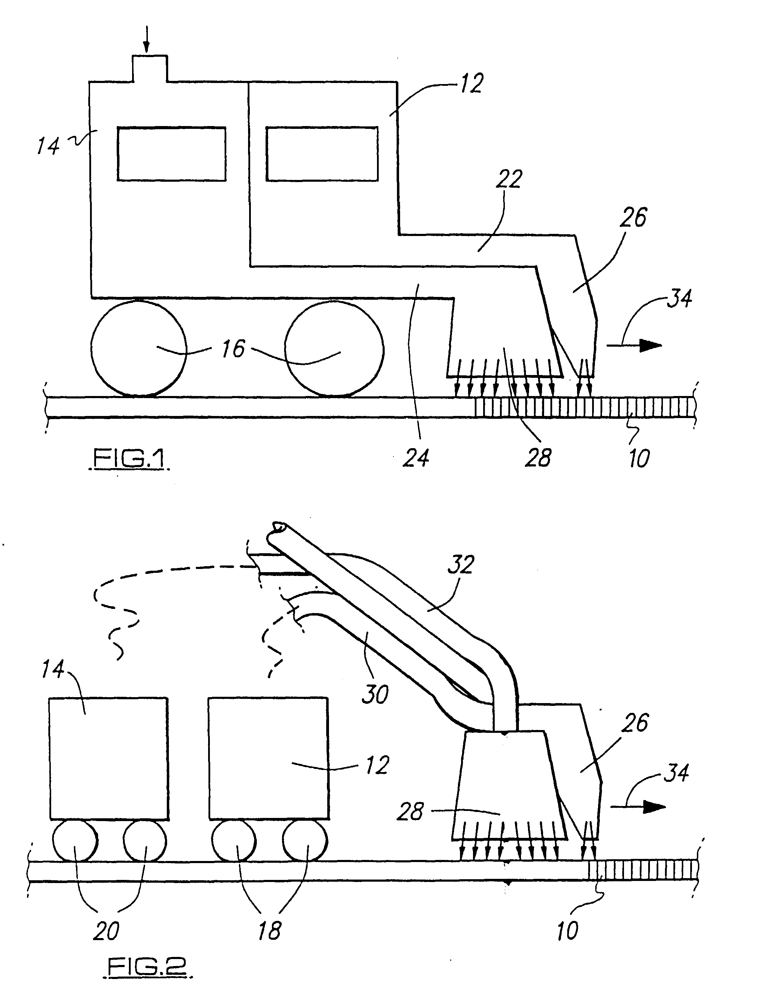

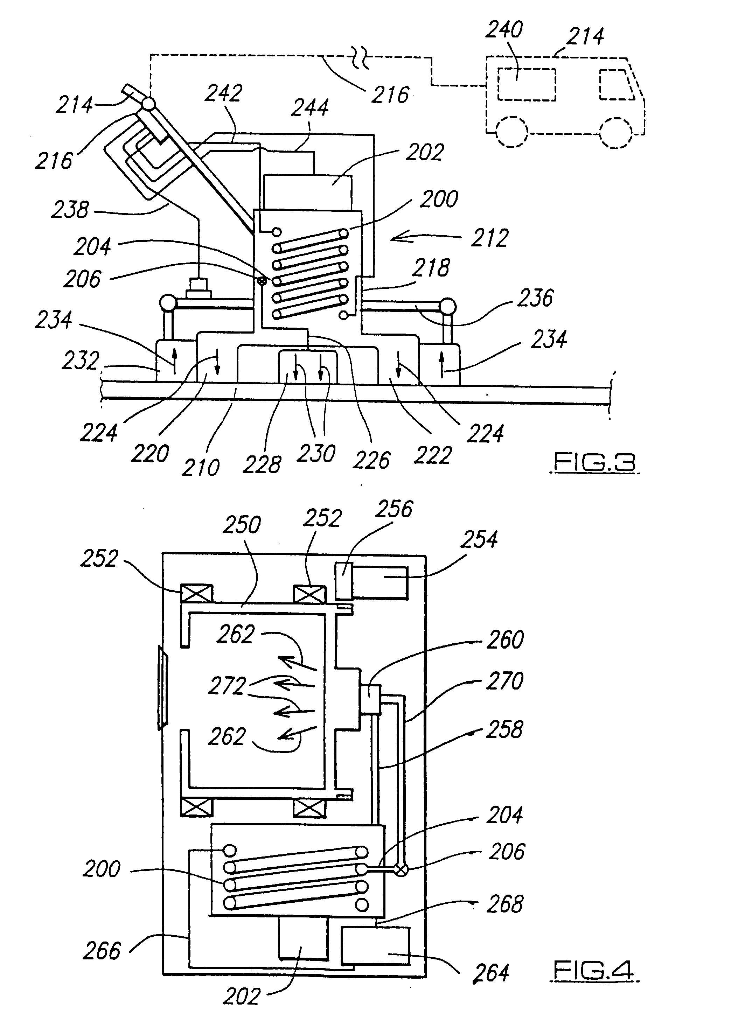

Referring to the drawings, and firstly to Figs. 1 and 2, these diagrams show apparatus when in use for treating a textile sheet material, in this case a carpet 10. The apparatus comprises a steam generating unit 12 and a hot air generating unit 14. In Fig. 1, the two units 12 and 14 are carried by a single chassis represented by wheels 16 whereas in the case of 2, these units 12 and 14 are carried by their own chassis represented by wheels 18 and 20.

In Fig. 1, ducts 22 and 24 connect the units 12 and 14 to outlet nozzles 26 and 28 which are arranged to jet the respective materials onto the carpet as indicated by the arrows. Nozzle 26 is relatively narrow and directs a jet of steam onto the carpet 10, whilst nozzle 28 is relatively broad and directs hot air onto the carpet. The basis of this operation is that the hot air should be applied for a longer period in order effectively to remove residual moisture created by the steam nozzle 26. The arrangement of Fig. 2 is similar to Fig. 1 except that the units 12 and 14 are connected by flexible conduits 30 and 32 to the nozzles 26 and 28.

To use the equipment shown in Figs. 1 and 2, it is simply a matter of propelling the combined nozzle head 26/28 over the carpet to be treated as indicated by the arrow 34. The manner in which the combined nozzle is moved is entirely at the option of the user, and in many cases will be performed by a contractor who will visit the location of the material to be treated and perform the treatment. The contractor will know the speed and frequency of application in order to effect the treatment, but it is preferred that the carpet be heated by the steam up to at least 100°C, and for a sufficient length of time to de-nature the allergens. The steam and hot air nozzles may be separate and may be on separate machines and may be performed separately by different operators, and equally after the hot air treatment it is preferably that the carpet be vacuum cleaned to remove the residues and particles.

The carpet or other article may be pre-heated using the nozzle 28 with the steam supply switched off, and other variations are possible.

Instead of using hot air for the force drying, other drying methods may be used including the use of a spray drying agent, but the important aspect of this invention is the utilisation of moisture preferably hot water for steam treatment or de-naturing the HDM allergen followed by the force drying step.

Figs. 3 and 4 show further embodiments of apparatus in accordance with aspects of the present invention. In each of the apparatus shown in Figs. 3 and 4, there is a steam driven heat exchange coil 200, the purpose of which is to heat air which is driven over the coil by means of an air blower 202. The hot air which flows from the heat exchange coil 200 is used for the heating of the material or article being treated for the killing of the HDM and for the de-naturing of the HDM allergen. In each case, there is a bleed off connection 204 whereby steam may be bled from the coil 200 selectively, and supplied through a control valve 206 to the article being treated in order to provide the moisture to be supplied to the article for the purposes as explained herein. The operation of the control valve 206 may be under manual control, or it may be under automatic control based upon a preprogramming of the equipment depending upon the material or article being treated. In general terms therefore when the article of material is to be treated, steam is applied thereto, and hot air is available of force drying of residual moisture created by the steam in the material or article. The hot air may be used for preheating the material or article, and it may be supplied in conjunction with the steam all at the control of the operator or depending upon the preprogramming arrangement. The following description will now explain the different modes of operation of the respective apparatus of Figs. 3 and 4.

In Fig. 3, the apparatus is for the treatment of sheet material such as a carpet 210. The steam heating coil is contained in a mobile head 212, which is connected to for example a vehicle 214 by means of an umbilical cord 216 which contains the necessary service pipe connections.

The head 212 is manipulable by an operator using a handle 214, and in this embodiment adjacent the handle 214 is a control box 216 by which the operator can control the operation of the equipment in the manner described below.

As shown in Fig. 3, the steam coil 200 is located in a casing 218 and the blower 212 is arranged to blow air over the steam coil and into hot air outlets 220 and 222 so that the hot air will be jetted onto the carpet 210 as indicated by arrows 224.

The outlet from the valve 206 is connected by pipe 226 to steam outlet nozzle 228 which lies between the hot air outlets 220 and 224 whereby steam may be jetted onto the carpet 210 as indicated by arrows 230.

Finally, surrounding the hot air outlets 220 and 222 is a vacuum ring 232 by which the materials may be sucked from the carpet 210 as indicated by arrows 234. The air and any excess steam sucked up by the ring 232 is charged into a collection ring 236, and eventually is discharged through discharge pipe 238 which leads to a valve in control box 216 whereby the control of the exhaust is under the operation of the operator.

Operation of the equipment will be understood from the description given above, and the nature of the invention as explained herein, but basically, a contractor would arrive with the vehicle 214 containing the head 212, and the head would then be used for the cleaning of carpets, curtains and the like in the customers premises, the vehicle 214 being provided with a suitable steam generator 240 to supply the steam to the coil through the umbilical coupling 216 and the circulation pipes 242 and 244 which lead to the control box 216. To treat the carpet, the operator controls the supply of steam and hot air as appropriate which may comprise applying hot air initially to the carpet, followed by the application of steam, followed by the application of hot air, the vacuum ring 232 being operating continuously during this period. The vacuum may be created by suction from the vehicle 214, the vehicle being provided with a suitable vacuum generator for this purpose, or it may be created by a separate blower on the head 212 connected to an adjacent power point. Equally, the blower 212 may be driven by power from an adjacent socket, in a similar manner to a vacuum cleaner.

In the equipment shown in Fig. 4, there is a tumble drum 250 supported by bearings 252 and the equipment is indeed a tumble dryer type apparatus. The drum 250 may be driven by means of an electric motor 254 and gearing 256 connected to drum 250.

The steam driven heat exchange coil 200 is located under the drum, and blower 202 serves to blow hot air over the heat exchanger coil 200 to be heated thereby, and hot air can then be injected into the drum 250 through pipe 258 and a rotary coupling 260 as indicated by arrows 262. Steam generator 264 is also included in the equipment, and is coupled to the coils 200 by circulation pipes 266 and 268. The outlet of the control valve 206 is connected to a pipe 270 which is also connected through a rotary coupling 260 so as to be able to charge steam into the drum as indicated by arrows 272.

With the equipment of Fig. 4, the same operational possibilities as the equipment of Fig. 3 can be realized. Hot air and/or steam can be injected selectively into the drum in order to treat an article which is placed in the drum. Such an article may be for example a duvet or a pillow, which can be initially heated by the hot air, and then can be treated with steam alone or steam and hot air together, followed by frying of the residual moisture on the article using the hot air. The equipment of Fig. 4 is self contained in nature and may be provided at a location attended by the general public for treatment of their articles, and therefore the equipment can be preprogrammed and coin operated to ensure that effective treatment takes place.

Figs. 3 and 4 are of course diagrammatic representations and are provided to indicate only the basic elements of the equipment. It is appreciated that the design configuration may be different from that indicated in these drawings, in order to suit any particular installation.

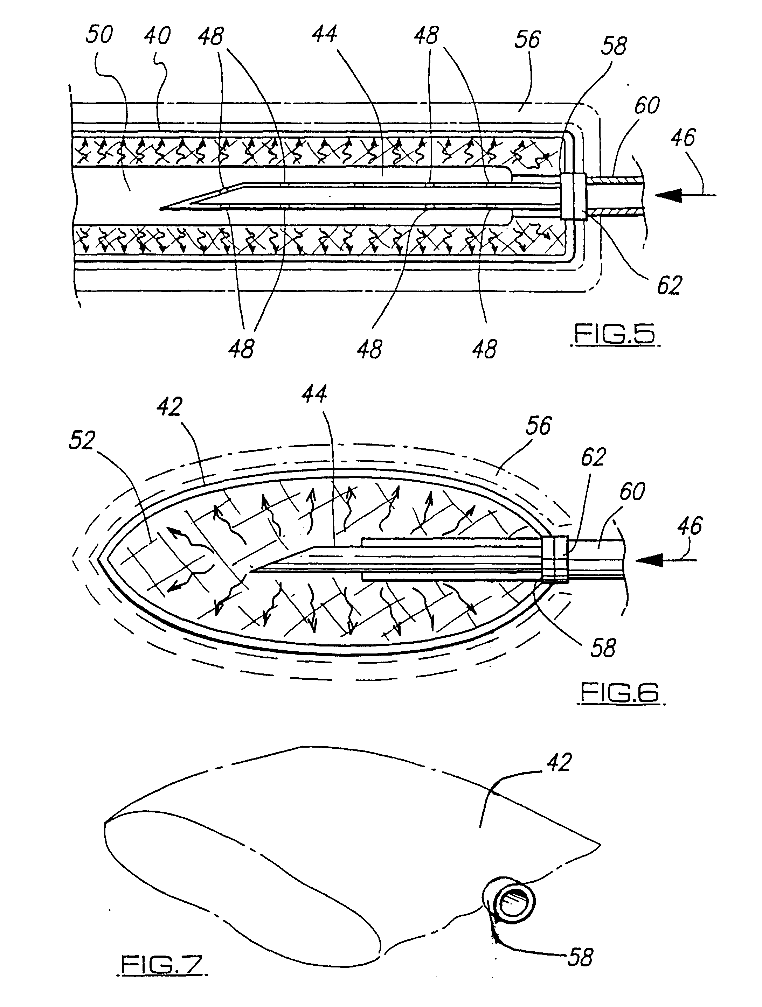

Figs. 5 and 6 illustrate the approach according to the embodiments of the second aspect of the invention. In Fig. 5 a mattress to be treated is indicated by reference 40, whilst reference 42 in Fig. 6 illustrates a pillow to be treated. In each case, an injection lance 44 projects into the core of the article, and hot fluid is injected into the lance as indicated by arrow 46 so as to heat the article from the core. The hot fluid injected into the lance 46 issues from the lance inside the article through outlets 48 and the hot fluid permeates from the core to the outer surface of the article, heating and treating same as it thus permeates.

In the case of the mattress of Fig. 5, it is shown with a hollow interior 50, but in the case of the pillow, it is shown as being filled with pillow filling 52.

Also shown in Figs. 5 and 6 is the provision of a heat retaining covering 56 which is wrapped around the article as it is treated, in order to retain the heat. The pillow and mattress is each provided with a special coupling 58 by which it can be readily coupled to the lance supply pipe 60, which is provided with a co-operating coupling 62. In use therefore it is simply a matter of bringing the lance 44 to the article and by pushing it into the core of the article followed by establishment of the coupling 58/62 which may be of the quick release variety. The hot fluid is then injected into the interior of the article to treat same. In a preferred method, hot air is first injected to pre-heat the article, and then steam is injected for the heating and moistening of the article, followed by the injection of hot air and therefore this aspect of the invention can use the first aspect of the invention described above.

However, the approach of providing heating from the core of the article has not previously been suggested in the prior art, and therefore this aspect may be designed in various different embodiments. For example, instead of using steam it may be possible to achieve an appropriate effect by the use of hot air only.

Additionally, the hot air may be provided by providing the article with an integral internal hot air blower which can be switched on selectively. Furthermore, the interior of the article may be provided with a heating element similar to an electric blanket.

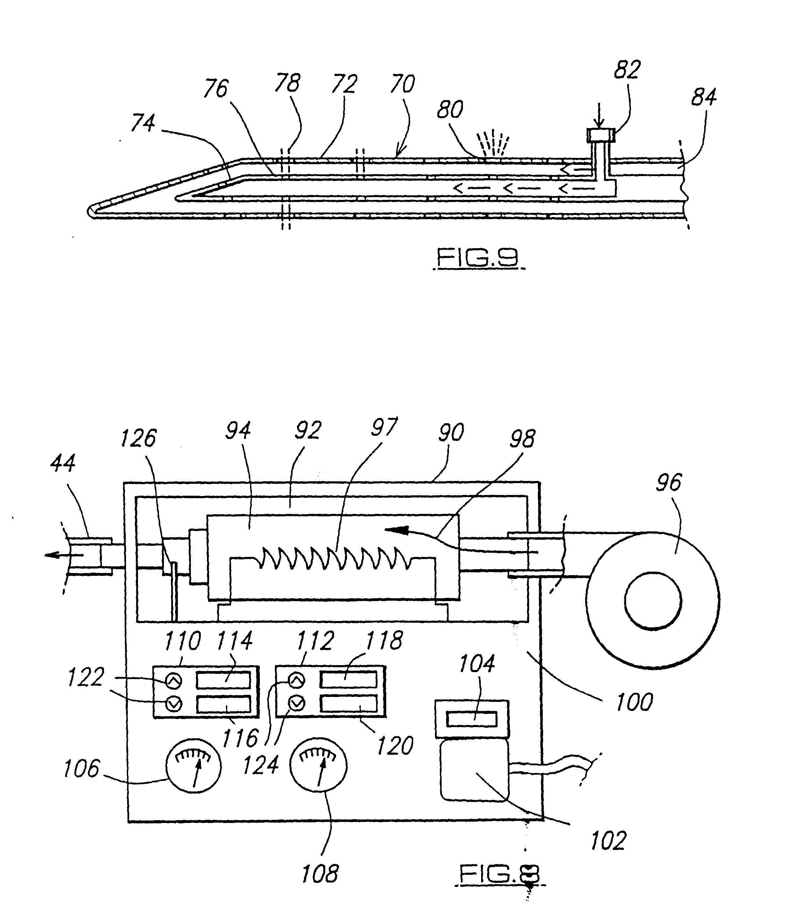

Fig. 7 shows the pillow of Fig. 6 in perspective view, and the coupling 58 which is integral with the pillow is clearly shown. An article provided with this special adaptation is of particular advantage, and constitutes a third aspect of the invention, because it is enivsaged that in the provision of the service of de-naturing HDM allergen and eliminating the HDM will involve the provision of not only the equipment for effecting the treatment, but also the specially adapted articles to be used by sufferers. An asthma sufferer therefore may have his bed, pillows, cushions, seats, sofas provided with special adaptation and construction to enable a contractor to come and to treat the articles for achieving the appropriate effect as described herein. Indeed, it may be possible to provide each sufferer with his own treatment equipment so that he can perform the treatment on his or her own.

Fig. 9 shows that a specially adapted lance may be used for the injection of the steam and the hot air rather than using separate lances. In the arrangement of Fig. 9, the lance 70 comprises an outer tube 72 from which the hot air issues, and an inner tube 74 from which the steam issues. The inner tube 74 has small jetting apertures 76 from which the steam issues as indicated by reference 78, and these apertures are in alignment with larger air outlets 80 in the tube 72. The steam inlet 82 is connected to the inner tube, whilst the air inlet is connected at region 84 to the hot air generator as will be explained in relation to Fig. 8.

Referring now to Fig. 8, this figure illustrates diagrammatically an equipment for the injection of hot air into an article as shown in Fig. 5 or Fig. 6.

The equipment comprises a casing 90 which typically would be of a size capable of being carried by one man. The casing has a cavity 92 in which is contained a process heater 94 for heating the air which is driven therethrough by means of a blower 96. This blower may be detachable for transportation. The cold air blown by the blower 96 passes as indicated by arrow 98 over the resistance heater 97 contained in the heater 94, to be heated thereby, and is driven into the lance 44 and eventually is distributed throughout the article as shown in Fig. 5 or Fig. 6.

Reference numeral 100 represents the portion of the casing which contains the controls, and an electrical supply is connected thereto as indicated by reference 102, which is controlled by switch 104. Dial 106 is an ammeter to show the current being drawn, whilst dial 108 shows a pressure gauge which indicates that the pressure of the air being supplied to the interior of the article.

The sub panels 110 and 112 represent indicators for selected temperature in display 114 and actual temperature in display 116 on the one hand, and selected time display 118 and actual time display 120 on the other hand. These units have up and down setting buttons 122 and 124 by which the set temperature and set time in displays 114 and 118 can be adjusted.

When the system is running, the temperature of the air sensed by thermocouple 126 will be displayed in display 116, and the elapsed time of the treatment will be indicated in display 120.

The equipment is controlled insofar as if there is a loss of pressure in the process heater 94, the heating element 97 is automatically switched off so that damage thereto will be avoided.

The time of each treatment will be varied, but it has been indicated that in the order of ten minutes at least will be required for the steaming of the article, when steam is supplied, and ten minutes heating will be required in order to dry off the moisture left from the steam treatment.

The process heater may have a power rating in the order of 5,500 watts for use with double bed mattresses, although a lower rating, perhaps in the order of 3,000 watts may be suitable for the mattresses for a single bed.

Instead of the lance being provided with a coupling and the article being adapted for receipt of the lance, the lance may simply be used to puncture an existing conventional article to be treated, and the puncture duly closed and repaired following treatment.

Where the article is encased in insulation, that insulation may cover the article fully except for in an area to receive the lance, and the covering may comprise a heating element for applying heat to the outer surface of the article.

The heating of the article is in effect causing all of the article to be raised to elevated temperature for the effective treatment thereof.

Using hot air it is possible to heat the article to a temperature such that the outer surface of the article reaches 120°C. This temperature was maintained for 5 to 10 minutes.

The insulating sheets may be air permeable and air outlet apertures may be provided in the sheath to promote the permeation of the heated air through the article being treated. On the other hand, the sheath may be air impermeable but be provided with an air outlet to allow the air to escape through such outlet, and such outlet can be coupled to the blower in order that the heated air will be re-circulated.

The heating may be thermostatically controlled by known methods in addition to or in place of the pre-setting of the temperature as described in relation to the apparatus in Fig. 8.

The sheathing of the article being heated may be varied in order to achieve best flow of the heating fluid therethrough. Thus, the size of a mattress may be sealed by means of sealing tape.

Instead of there being one lance injected into the article to be treated, where appropriate, there may be several of said lances coupled to a common source of supply of hot fluid.

A major practical application of the invention is the effective treatment of mattresses; no prior method has effectively addressed the difficulty of effectively treating mattresses for the elimination of the HDM and the de-naturing of the HDM allergens. All prior treatments have been surface treatment suggestions which are unlikely to be effective in failing to give sufficient penetration for treating the deeper lying layers.

A mattress may consist of a steel frame, resilient steel springs, textile paddings respectively wrapped over on the upper and lower regions of the frame but not extending to an intermediate region around the periphery of the mattress, and an outer fabric or textile covering. Interiorly of the sprung frame may be an air-filled cavity. The textile paddings of a mattress commonly consist of varous layers to increase the comfort of the mattresses, often including layers of horse-hair matting and layers of synthetic material such as nylon. All of these textile layers are liable to become infested with HDM through time, in particular those on the upper padding where the largest quantities of shed skin collect since it is on the upper surface that a person sleeps, continually shedding skin fragments which penetrate deeper and deeper into the upper mattress padding, and then onto the lower padding, and the skin particles may eventually penetrate the lower padding and become deposited onto the bed base. Thus, both mattresses and bed bases are potential sources of the HDM allergen.

It is to be mentioned that the use of an insulating sheath 22 may not be necessary if a sufficient temperature can be achieved without the sheath. It may also be possible to achieve the required temperatures for example by the use of an insulating substantially air-impervious insulating strip surrounding the sides of the mattress, from which most of the hot air would otherwise escape rather than permeating through the padded fabric regions. This insulating strip would allow an aperture similar to aperture 24 in sheath 22 to provide access for the air blower head 20 to the side of the mattress cover 14. Whichever means of insulating is used, if indeed any at all is used , it is preferred that all of the mattress fabrics, including the paddings 8 and 10 and the cover 14, can be effectively elevated in temperature in order to substantially eradicate the HDM and its allergen from those fabrics in a single treatment of the mattress. In order to de-nature the HDM allergen moisture, preferably in the form of steam, should be supplied in combination with heated air or alone, and throughout the mattress materials.

By adopting an arrangement whereby the hot gases are effectively distributed throughout the fabric-containing portions of the article from the core thereof, the required temperature conditions can be achieved thoroughout the fabrics of the mattress. As the heated gas or gases permeates throughout the body of the pocket-sprung mattress it moistures and elevates the temperature thereof sufficiently to kill and the HDM/de-nature the HDM allergen as required.

In order to kill the HDM the temperature of the fabric and the gas permeating through the fabric must be sufficiently elevated for a sufficient period of time. Furthermore, in order to de-nature the HDM allergen, sufficient moisture must be supplied in combination with the heat, and to provide an effective service, the article must be force dried if steam is used. If a relatively low elevated temperature is used, the elevated temperature must be maintained over a longer period of time than that required at higher tempertures.

In a series of experiments which we have carried out, reductions in levels of the HDM allergen (Der.p1) implanted in a mattress were measured after various treatments with steam and hot air. The hot air and steam were injected directly into the body of the mattress by means of a 3.5 kW process heater and a 3 kW domestic steamer respectively.

In the first experiment, steam alone was injected over a period of 20 minutes. In the second, a hot air and steam combination and then hot air only was injected each for 10 minutes respectively. In the third, steam and hot air were supplied consecutively over a period of 15 minutes. The fourth experiment involved hot air alone, followed by steam alone, and followed by hot air alone for a period of five minutes each. In the fifth experiment, hot air and steam were supplied in combination for a period of 15 minutes. Finally, the HDM allergen was incubated in a dry state in a hot air oven at 120° for 15 minutes.

It was found that reductions of generally at least 90%, and in one case, 99.9%, were obtained in the levels of allergen at the test sites in the mattress in respect of the first five experimental methods all involving steam. In contrast, the dry treatment in the hot air oven resulted in no measurable decrease in the levels of HDM allergen over the unheated control sample.

The treatment of a mattress or other article containing fabric may be more time consuming than these indicated times, since additional time should be allowed for elevating the temperature throughout the materials of the article to the required temperature. The use of permeating hot gas ensures that this additional time requirement may be kept to a minimum and the effectiveness of the process is maximised so as to readily destroy the HDM and its allergen throughout the fabric materials.

Where the article is large and well insulated, the article may be injected with gas initially at one rate of calorific delivery for a first period of time in order to heat the article to the desired temperature, and subsequently the gas may be injected at a lower calorific rate in order to maintain the desired temperature. This procedure ensures that treatment may be achieved in as little time as possible whilst conserving energy.

The above description is not to be construed as an exhaustive definition of the present invention. Rather it will be appreciated by a man skilled in the art that the features of the various embodiments may be combined and utilised in conjunction with one another. Various modifications may be made using equivalent procedures and/or equipment whilst remaining within the scope of the present invention.

Furthermore, where the article is for example a sheet material such as carpet, the application of water which may be initially cold but is then heated to a temperature approaching 100°C, at least 60°C, may be effective to eradicate the HDM allergen. The time required for this treatment example is expected to be longer, and water removal after treatment will be required.

Thus, the disclosure of features described in relation to any one embodiment of the invention should be taken as a disclosure of those features used in any other embodiment, insofar as the features are compatible with that embodiment.

The invention provides an effective method and means for the de-naturing of HDM allergens, and the various steps employed also address the matter of extermination of the HDM itself although, as indicated herein, it is the HDM allergens which are the major cause of discomfort in persons with respiratory problems.

Of particular significance is the utilisation of heat in conjunction with moisture either in the form of water or steam for the de-naturing of the allergens as it has been found that it is necessary to use moisture to make the de-naturing effective as indicated by the tests described herein.

In practise, because the steam is applied mainly to articles and materials in situ, when the steam is applied to the material or article it will be at atmospheric pressure, which in turn means that in practise it will not be capable of heating the article using the steam to more than 100°C, but fortunately, such a temperature is adequate to de-nature the allergen and kill the HDM. In the invention, it is preferred that the material or article be heated to a temperature in the order of 100°C, and especially in the case of the 3-dimensional articles referred to herein such as pillows, mattresses, cushions, soft toys and the like. If the whole article is to be heated from its core up to a temperature of 100°, this may require the use of an insulating sheathing to prevent heat loss, but there will be effective and complete de-naturing of the allergens and extermination of the house dust mite.

It can be seen therefore that the invention provides a means whereby, for example, a servicing company can visit domestic dwellings and other locations in order to perform the de-naturing and elimination of HDM allergen and HDM. Such a service can be carried out on a periodic basis providing for example asthma sufferers with a continuing HDM and HDM allergen free environment, which will be extremely important to and provide great relief for asthma sufferers.

The process is made particularly effective if the articles to be treated in this way are custom designed so as to have an integral coupling and conduit means leading to the core of the article, and such coupling is designed to intercouple with an outlet of portable equipment which the contractor who will perform the service brings with him when he comes to treat the articles in a household.

The invention therefore also provides a combined set of equipment and articles to be treated, the articles to be treated being articles of domestic use such as pillows and mattresses which remain in situ in each customer's dwelling, whereas the portable apparatus which is designed to intercouple with the articles is the unit which is retained by the contractor. It can be seen therefore that a network of equipment located throughout the country can be used for performing a de-naturing service on articles in domestic dwellings. Indeed, in some cases it may be appropriate to design and produce a set of equipment which is for retention in a household so that the owner can perform the de-naturing and HDM elimination on his own. An excellent new and advantageous service is created by the invention which will provide extended benefit to people with respiratory problems.

Our work has shown that when moisure is used for the de-naturing and HDM elimination process, the use of drying of the residual moisture enhances the operation, and is particulary convenient in enabling the process to be completed as soon as possible. It is desirable therefore that after the moisture treatment, the article or material is dried using heated gas, and in particular hot air.

In the case of treating an article as described, the hot air preferably is injected into the core of the article (although other methods are possible as described herein) and the air, like the steam before it heats the article from the core, and dries off any residual moisture. The air like the steam moves outwardly through the article to the surface thereof.

When the invention is used for the treatment of a sheet material such as a carpet, curtain or upholstery fabric on the outside of an article, it is preferred that a combined machine be provided which has an outlet nozzle for steam, and an outlet nozzle for hot air, and the nozzle is moved over the sheet material in a direction whereby the steam is applied first to the material and this is followed by the application of hot air. The hot air outlet nozzle will probably have to be bigger than the steam nozzle in order to provide effective drying off of residual moisture and in order to ensure that the hot air will be applied for a longer period than the period of application of the steam.

The invention provides a solution to a problem which is in fact endemic in the United Kingdom and is on the increase. The problem also exists in other countries throughout the world.

## Claims

1. The use of moisture and heat for denaturing of HDM allergen wherein moisture in the form of steam is applied directly to textile materials such as curtains, carpets, upholstery fabrics and mattress and divan fabrics whilst in situ, for a period to de-nature the HDM allergen, followed by the force drying of the fabrics whilst in situ to remove any residual moisture.

2. The use according to claim 1, wherein the steam is applied at a temperature of at least 80 degrees C.

3. The use according to claim 1 or 2, wherein the forced drying is by means of hot air.

4. The use according to claim 3, wherein the hot air is blown at the textile material.

5. The use according to claim 2 or 3, wherein the hot air is applied to the textile material at a temperature of at least 80 degrees C.

6. The use according to any preceding claim, wherein the forced drying is carried out for a period of at least 5 minutes.

7. The use according to any preceding claim, including the step of applying heat to the textile material whilst in situ before the application of the moisture.
